# EUROPEAN PATENT SPECIFICATION

(11) **EP 0 644 751 B1**
(45) Date of publication and mention of the grant of the patent: **03.09.1997**
(21) Application number: 93913340.1
(22) Date of filing: 10.06.1993
(51) Int. Cl.: A61K 7/16

(54) **MOUTHWASH FOR TREATING DENTINE HYPERSENSITIVITY**
MUNDWASSER ZUR BEHANDLUNG DER DENTINHYPEREMPFINDLICHKEIT
COLLUTOIRE POUR TRAITER L'HYPERSENSIBILITE DE LA DENTINE

(30) Priority: 16.06.1992 GB 9212706
(43) Date of publication of application: 29.03.1995
(73) Proprietor: SMITHKLINE BEECHAM PLC, Brentford, Middlesex TW8 9EP (GB)
(72) Inventor: NISBET, Mark Andrew, SmithKline Beecham, Weybridge, Surrey KT13D0DE (GB); GRIST, Nigel James, SmithKline Beecham, Weybridge, Surrey KT13 0DE (GB); SCOTT, Claire Michelle, SmithKline Beecham, Weybridge, Surrey KT13 0DE (GB)
(74) Representative: Walker, Ralph Francis, Dr.
(86) International application number: GB9301237
(87) International publication number: WO9325183

(56) References cited:
- EP-A- 0 200 323
- EP-A- 0 346 957
- EP-A- 0 390 456
- WO-A-92/07547
- FR-A- 1 600 227
- US-D- 3 863 006

## Description

The present invention relates to the use of mouthwash compositions comprising particulate solids for treating dentine hypersensitivity and to mouthwash compositions comprising certain particulate solids.

The occurrence of sensitivity in teeth is an increasingly common dental problem so that there is as continuing need not oily for improved actives but also improved presentations. Suitable actives known include water soluble strontium and potassium salts such as strontium acetate, strontium chloride, potassium nitrate, potassium chloride and potassium citrate; all of which are used in commercially available toothpastes. More recently, it has been suggested that an oral hygiene composition, in particular a dentifrice, comprising a combination of an insoluble agent (hydroxyapatite) and a soluble agent (potassium and/or strontium salt) may have useful antisensitivity properties. (EP 0 346 957-A, Unilever). Until recently, the oily antisensitivity compositions available were dentifrices. It is believed that in such dentifrices, the abrasive may also play a role, by occluding tubules and thereby providing an anti-sensitivity effect.

Recently, there has been an upsurge of interest in the concept of antisensitivity mouthwashes. There is for instance now available in the UK, from Boots, a mouthwash containing potassium citrate which is alleged to be of use in treating sensitive teeth. In addition, there has been recently disclosed an abrasive mouthwash composition, for use as a "rinse and brush" product. This may optionally include a therapeutic agent, for instance an anti-plaque, anti-tartar or anti-sensitivity agent (WO 92/07547). EP-A-0 200 323 and EP-A-0 390 456 disclose oral hygiene compositions such as mouthwashes for treating dentine hypersensitivity comprising a strontium salt, a fluoride source and and abrasive and/or thickening silica. It is not known, however, that the abrasive and/or non abrasive particles themselves are able to alleviate dentine hypersensitivity.

We have now surprisingly found that finely divided solids when delivered in a mouthwash composition are effective in alleviating dentine hypersensitivity.

The present invention therefore provides for the use of an anti-sensitivity system consisting of finely divided particulate solids and a water-soluble anti-sensitivity agent in the manufacture of a mouthwash composition further comprising a dentally acceptable vehicle or carrier, for treating dentine hypersensitivity.

Finely divided particulate solids for use in the present invention may be abrasive or non-abrasive. In general, abrasive particles tend to have a larger particle size. Mixtures of solids, including mixtures of abrasive and non-abrasive solids may be used. The incorporation of an abrasive solid will provide the composition with an element of tooth cleansing power.

In one embodiment, the present invention provides for the use of an antisensitivity system which consists of finely divided abrasive and/or non-abrasive particulate solids in the manufacture of a mouthwash further comprising a dentally acceptable vehicle or carrier, for treating dentine hypersensitivity.

Suitable non-abrasive particulate solids include non-abrasive silicas; non-abrasive polymer resins; non-abrasive chalks, such as Calopake F ™ (Sturge); andnonabrasive insoluble or sparingly soluble inorganic salts such as potassium lauryl sulphate or titanium dioxide. Mixtures of different particulate solids may be used.

Suitable non-abrasive silicas include manufactured silicas, for instance fumed silicas, such as Aerosil 200™ (Degussa); silica gels and precipitated silicas, such as Syloblanc 34 ™ (Grace), Sorbosil TC25 ™ (Crosfield), Tixosil 43 ™ (Rhone-Poulenc) and Sylodex ™ (Grace); and naturally occurring silicas, such as diatomaceous earths. A preferred silica for use herein is Syloblanc 34 ™.

Suitable abrasive solids include those conventionally used as dentifrice abrasives, including silica, calcium carbonate, alumina, calcium phosphates including dicalcium phosphate dihydrate and calcium pyrophosphate, insoluble sodium metaphosphate and synthetic plastic resins.

Suitable abrasive silicas include natural amorphous silicas such as diatamaceous earth and synthetic amorphous silicas such as silica gels and precipitated silicas. Suitable silica xerogels are described in US patent no. 3 538 230. Preferred preciptated silicas are those available under the trade marks Zeodent and Tixosil, by J M Huber Corporation and Rhone-Poulenc, respectively.

Other suitable finely divided particulate solids for use in the present invention include other insoluble or sparingly soluble dentally acceptable inorganic salts such as insoluble zinc salts, for instance, zinc phosphate and zinc hydroxystannate; insoluble strontium salts, for instance, strontium carbonate and strontium sulphate,; and magnesium salts, for instance, magnesium silicate and magnesium phosphate.

Suitably, when the antisensitivity system comprises a mixture of non-abrasive and abrasive solids in combination with a water soluble anti-sensitivity agent, the ratio of non-abrasive to abrasive particulate solids is from 100:1 to 1:2, preferably 50:1 to 1:1, more preferably 10:1 to 2:1. by weight..

Suitably the particulate solid is present in a range of from 1 to 15%, preferably 2 to 10%, more preferably 2 to 8% by weight of the mouthwash.

Suitable water soluble antisensitivity agents are well known in the art and include water soluble strontium salts, water soluble potassium salts and mixtures thereof.

Suitable water soluble potassium salts are well known in the art and include the nitrate, chloride, bromide, iodide, citrate, acetate, lactate and hydrogen carbonate, of which the nitrate, chloride, citrate and acetate are preferred. Typically, the potassium salt will be present in a range to provide from 0.01 to 5%, preferably 0.05 to 3% by weight of the composition as potassium ions.

Suitable water soluble strontium salts are well known in the art and include the chloride, bromide, iodide, acetate, edetate, nitrate, salicylate and lactate, of which the chloride and acetate are preferred. Typically, the strontium salt is present in a range to provide from 0.01 to 5%, preferably 0.05 to 3% weight of the composition as strontium ions.

Suitable ionic fluorine-containing compounds are well known in the art and include fluoride salts, such as the alkali metal fluorides, for instance sodium fluoride, ammonium fluoride, amine fluorides, tin (II) fluoride and zinc fluoride, of which sodium fluoride is preferred. In addition to, or instead of the above fluoride salts, the ionic fluorine-containing compound may also be a monofluorophosphate salt, preferably an alkali metal monofluorophosphate such as sodium monofluorophosphate or a mono-fluoropolyphosphate salts, for instance a compound of the formula Na₄P₃O₉F, K₄P₃O₉F, Li₄P₃O₉F, Na₃KP₃O₉F or (NH₄)₃NaP₃O₉F. Typically the ionic-fluorine compound will be present in an amount to provide from 50 to 1000 ppm, preferably from 100 to 500 ppm of fluoride ions.

Composition for use in the present invention may also optionally comprise other agents known to enhance the anti-caries effect of fluoride and monofluorophosphate. Thus, for instance calcium glycerophosphate is known to enhance the anti-caries efficacy of monofluorophosphate and may be incorporated in a weight ratio of up to 1:3, preferably 1:20 to 1:3, compared to the weight of monofluorophosphate.

It will be appreciated that strontium ions may be incompatible with the presence of fluoride ions, due to the formation of insoluble strontium fluoride, so that such a combination of antisensitivity and anti-caries agent should be avoided.

Compositions for use in the present invention may further comprise an antiplaque agent, to enhance the overall profile of the composition. Suitable antiplaque agents are well know in the art and include cationic antibacterial agents such as a *bis*-biguanide, for instance chlorhexidine or alexidine and water-soluble salts thereof; quaternary ammonium compounds, for instance cetyl pyridinium chloride, hexitidine, benzthonium chloride, octenidine and hexamidine; non-cationic antibacterial agents, for instance triclosan and bromochlorophene and zinc salts, for instance zinc citrate. Typically the antiplaque agent will be present in from 0.005 to 1%, preferably 0.01 to 0.5% by weight of the composition.

Compositions for use in the present invention may also include a water-soluble film-forming polymer, such as the sustained release acrylic polymer-based systems described in EP 0 404 558-A (Yissum Research Development Company of the Hebrew University of Jerusalem). These will form a thin film on the exposed dentine surface, thereby entrapping particulate solids and watersoluble anti-sensitivity agents.

The dentally acceptable excipient or carrier will typically comprise a liquid vehicle which comprises water, preferably in combination with a further solvent such as ethanol.

The carrier may also comprise a surfactant which may be an anionic or a nonionic surfactant.

Suitable water-soluble non-soap or synthetic organic detergents for use herein include the water-soluble salts of higher fatty acid monoglyceride monosulphates (such as sodium hydrogenated coconut fatty acid monoglyceride monosulphate); higher alkyl sulphates (such as sodium lauryl sulphate); alkyarylsulphonates (such as sodium dodecylbenzenesulphonates); and the higher alkylsulphoacetates (for example sodium lauryl sulphoacetate). Also useful are the fatty acid amides in which the amino acid portion is a (C₂₋₆)alkylmonomino carboxylic acid, such as the fatty acid amides of glycine, alanine, 3-aminopropanoic acid, valine, sarcosine derivatives (such as N-lauroyl, myristoyl and palmitoyl sarcosinate compounds), sodium laurylsulphate, and sodium dodecylbenzenesulphonate. Other suitable anionic surfactants include the low-anion surfactants, such as sodium *N*-methyl-*N*-cocoyl taurate which is marketed by Croda under the trade name Adinol CT ™.

Suitable nonionic surfactants include for example polyethoxylated sorbitol esters, in particular polyethoxylated sorbitol monoesters, for instance; PEG (40) sorbitan di-*iso*-stearate and Tween™ (ICI); polycondensates of ethylene oxide and propylene oxide (poloxamers), for instance Pluronic™ (BASF-Wyandotte); condensates of propylene glycol; polyethoxylated hydrogenated castor oil, for instance cremophors and sorbitan fatty esters.

The surfactant will typically be present in from 0.01 to 2%, preferably 0.05 to 1% by weight of the composition.

The dentally acceptable carrier may also usefully comprise one or more thickening agents such as derivatives of cellulose and carboxymethylcellulose, for example hydroxypropyl methyl cellulose, hydroxyethyl cellulose and Avicel ™ ; biopolymers, for example Chitin ™, Chitosan ™ (Protan); copolymers based upon pyrrolidione as one of a monomeric units, for example Gantrez (ISP); PVP/VA copolymers (ISP); alginates and derivitives thereof, for example propylene glycol alginate; xanthan gums and related polysaccharides; carrageenans and genuviaco TPH1; starch and derivatives thereof; polymers of acrylic and methacrylic acids, for example carbopols; inorganic minerals, for example veegum; natural gums, for example locust bean gum, guar gum and derivatives thereof, for example guar hydroxytrimonium chloride; gum arabic; colloidal polygalactosides, for example agar; synthetic hectorites, for example laponite; synthetic polymers, for example propylene oxide/ethylene oxide copolymers; and gelatin. The thickening agent will typically be present in a range from 0.1 to 2% by weight of the composition. It will be readily appreciated by the skilled man that the viscosity characteristics of compositions for use in the present invention may be adjusted to provide compositions in which the finely divided paticulate solids remain in suspension, semi-suspension or which more or less gradually settle down on standing.

The dentally acceptable carrier may also comprise humectants, such as glycerin, sorbital, xylitol, polyethylene glycol or propylene glycol. Additionally, the carrier may also comprise other adjuvants conventionally incorporated into mouthwash formulations, for instance colouring agents, flavourants, sweetening agents and preservatives.

Compositions for use in the present invention will have an orally acceptable pH which will typically be in the range pH 5.5 to 9.

Compositions for use in the present invention may be prepared by conventional procedures which include admixing the appropriate ingredients in the relative proportions in any order that is convenient and finally and if necessary adjusting the pH.

The compositions for use in the present invention may be provided in suitable containers in a "ready-to-use" form or as a more concentrated formulation which is then diluted by the user to the appropriate strength, prior to use. In addition, the various solid components may be provided as a dry powder which may then be made up into an aqueous composition by the user prior to use. Such dry powder may be usefully provided as an individual portion in a sachet, for greater convenience. The following examples illustrate the invention:

| **Example** | **1 w/w %** | **2 w/w %** | **3 w/w %** | **4 w/w %** |
|---|---|---|---|---|
| Ethanol | 15.00 | 15.00 | 15.00 | 15.00 |
| Tween 80 | 0.20 | 0.20 | 0.20 | 0.20 |
| Strontium acetate | 1.00 | 1.00 | 1.00 | 1.00 |
| Potassium nitrate | 0.63 | 0.63 | 0.63 | 0.63 |
| Sodium saccharin | 0.05 | 0.05 | 0.05 | 0.05 |
| Silica (Syloblanc 34) | 3.00 | 4.00 | 5.00 | 6.00 |
| Flavour | 0.10 | 0.10 | 0.10 | 0.10 |
| Water (de-ionised) | qs | qs | qs | qs |

| **Examples** | **5 w/w %** | **6 w/w %** | **7 w/w %** | **8 w/w %** |
|---|---|---|---|---|
| Ethanol | 15.00 | 15.00 | 15.00 | 15.00 |
| Tween 80 | 0.20 | 0.20 | 0.20 | 0.20 |
| Strontium acetate | 1.00 | 1.00 | 1.00 | 1.00 |
| Potassium nitrate | 0.63 | 0.63 | 0.63 | 0.63 |
| Sodium saccharin | 0.05 | 0.05 | 0.05 | 0.05 |
| Silica (Syloblanc 34) | 3.00 | 4.00 | 5.00 | 6.00 |
| Flavour | 0.10 | 0.10 | 0.10 | 0.10 |
| Xanthan Gum | 0.50 | 0.50 | 0.50 | 0.50 |
| Water (de-ionised) | qs | qs | qs | qs |

**Examples 9 to 12** - Mouthwashes may be prepared in a manner similar to that of Examples 5 to 8 but containing 0.25% rather than 0.5% xanthan gum (Keltrol F ™ ).
**Examples 13 to 16** - Mouthwashes were prepared in a manner similar to that of Example 1, containing potassium nitrate rather than potassium nitrate and strontium acetate and also sodium fluoride:

| **Examples** | **13 w/w %** | **14 w/w %** | **15 w/w %** | **16 w/w %** |
|---|---|---|---|---|
| Ethanol | 15.00 | 15.00 | 15.00 | 15.00 |
| Cremophor RH60 * | 0.20 | 0.20 | 0.20 | 0.20 |
| Potassium nitrate | 0.63 | 3.00 | 3.00 | 3.00 |
| Sodium saccharin | 0.05 | 0.05 | 0.05 | 0.05 |
| Silica (Syloblanc 34) | 3.00 | 3.00 | 3.00 | 3.00 |
| Xanthan gum ** | 0.25 | 0.25 | 0.25 | - |
| Sodium fluoride | 0.05 | 0.05 | 0.05 | 0.05 |
| Avicel Cl 611*** | - | - | - | 2.00 |
| Flavour | 0.10 | 0.10 | 0.10 | 0.10 |
| Glycerin | - | - | 25.00 | - |
| Water (de-ionised) | qs to 100 | qs to 100 | qs to 100 | qs to 100 |

| | | | | |
|---|---|---|---|---|
| * Cremophor RH60 (BASF) is a trade name for a PEG 30 hydrogenated caster oil product | | | | |
| ** Xanthan gum is Keltrol F ™ (Kelco) | | | | |
| *** Avicel Cl 611 (FMC) is a trade name for a mixture of microcryalline cellulose and cellulose gum | | | | |

**Example 17 -** The mouthwash formulations of Examples 1 to 4 were evaluated *in vitro* for tubule occulsion. Figures for presentation of tubule occlusion are based upon comparison with water controls.

### Method - Procedure for Evaluation of Tubule Occlusion by a Mouthwash Formulation

**(a) Preparation of Dentine Section -** Transverse circular slices are cut from the region of human teeth (3rd molars) lying between the enamel crown and the pulp cavity. Each slice is cut into 6 - 12 centrosymmetric sections comprising predominatly dentine with tubules running through the structure from one face of the segment to the other.
   The dentine sections (segments) are cleaned in sodium hypochlorite solution for 15 min., rinsed, etched with orthophosphoric acid for 5 mins and rinsed again. The dentine is allowed to dry briefly and mounted on 3/4 araldite stubs attached to removable supports. Two sections originating from the same tooth are mounted on each stub. Sections are assigned to treatment groups such that each treatment group contains the same number of sections from any given tooth.
   The sections are immersed (hand-held) in filtered, pooled, whole human saliva overnight to allow a pellicle coating to form.
**(b) Treatment of Dentine -** The section are immersed in a sample of stirred mouthwash for 1 minute, with the dentine facing into the vortex, then rised similarly in deionised water for about 15 seconds. One treatment group is assigned as a negative control and treated with water. The dentine is allowed to dry before gold coating prior to examination by Scanning Electron Microscopy.
**(c) Evaluation of Test Formulations -** Five randomly sectioned areas of dentine are chosen from different regions of each section. Using a fixed predetermined magnification the number of unoccluded tubules is counted in each area. Allowance is made for partially unoccluded tubules e.g. two halves equals one whole etc.
   Numerical evaluation can be supplemented by visual information as required. A series of photographs of randomly selected areas of dentine designed to be representative for each formulation, can be recorded for each test product.
**(d) Statistical Analysis -** Data generated consists of five tubule counts per section per treatment. These raw data are analysed by 1-way ANOVA with TUKEY to establish statistical significances between different treatments.

A figure for percentage tubule occlusion for each test product is derived from the average number of unoccluded tubules compared to the water control.

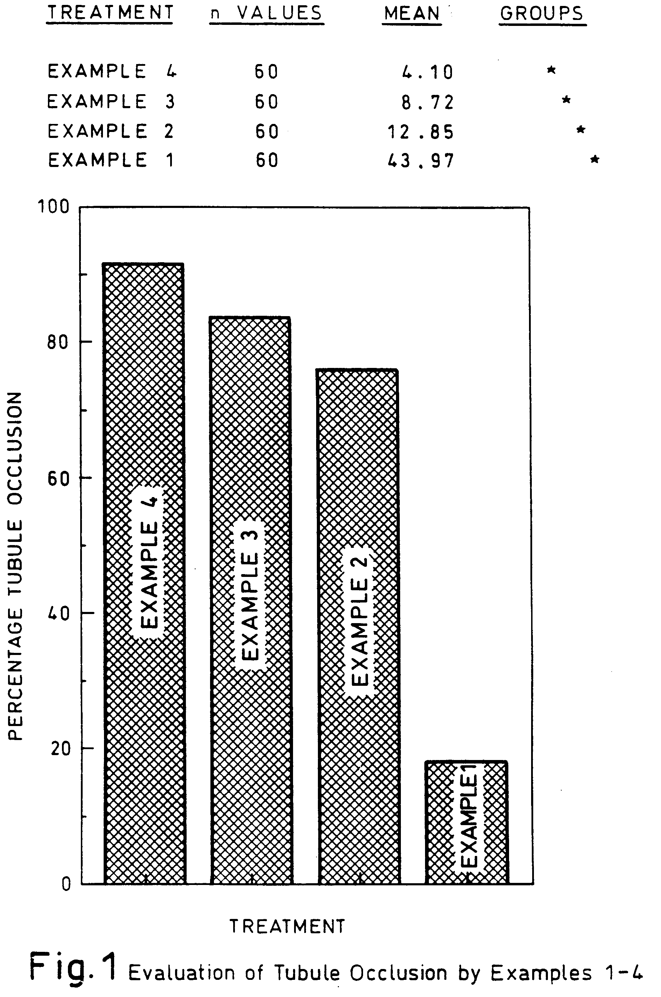

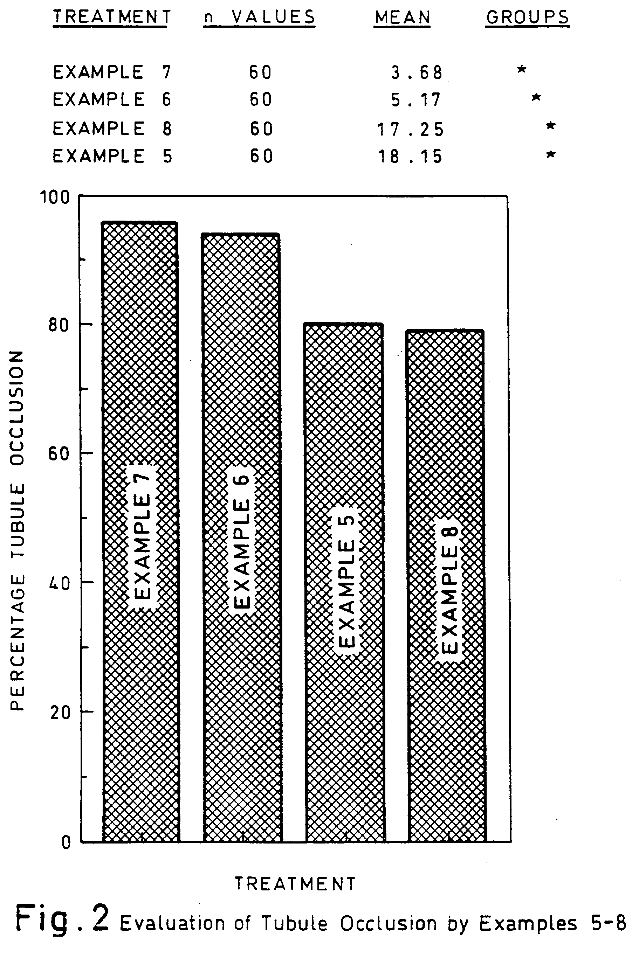

**Results -** The results are presented graphically in figure 1 and depict average tubule occlusion for each formulation, with statistical analysis by one-way ANOVA and then TUKEY. Asterisks (*) in different columns means that the results are significantly different (at the 95% level) whilst asterisks in the same column indicate a lack of a significant difference.
**Example 18 -** The mouthwashes of examples 5 to 8 were evaluated as in Example 17 and the results are presented graphically in figure 2.

## Claims

1. The use of an anti-sensitivity system which consists of finely divided abrasive and/or non abrasive particulate solids in the manufacture of a mouthwash composition further comprising a dentally acceptable vehicle or carrier, for treating dentine hypersensitivity.

2. The use of an anti-sensitivity system which consists of finely divided abrasive and/or non abrasive particulate solids and a water-soluble anti-sensitivity agent in the manufacture of a mouthwash composition further comprising a dentally acceptable vehicle or carrier, for treating dentine hypersensitivity.

3. A use as claimed in claim 1 or 2 in which the finely divided particulate solids are non-abrasive, optionally admixed with particulate solids which are abrasive.

4. A use as claimed in claim 3 in which the finely divided particulate solids are a non-abrasive silica.

5. A use as claimed in any one of claims 2 to 4 in which the water-soluble antisensitivity agent is a water soluble potassium or strontium salt or a mixture thereof.

6. A use as claimed in claim 5 in which the potassium salt is selected from potassium chloride, potassium nitrate, potassium citrate or potassium acetate.

7. A use as claimed in 5 in which the strontium salt is selected from strontium acetate or strontium chloride.

8. A use as claimed in any one of claims 1 to 7 in which the composition further comprises a fluoride ion source.

9. A use as claimed in any one of claims 1 to 8 in which the composition further comprises an anti-plaque agent.

10. A use as claimed in any one of claims 1 to 9 in which the composition further comprises a film-forming polymer.

11. A use as claimed in any one of claims 1 to 10 in which the composition further comprises a thickening agent.

## Patentansprüche

1. Verwendung eines Anti-Sensibilitätssystems bestehend aus feinverteilten schleifenden und/oder nicht schleifenden Feststoffteilchen zur Herstellung einer Mundwasserzusammensetzung, ferner umfassend einen zahnmedizinisch verträglichen Träger oder Vehikulum, zur Behandlung von Überempfindlichkeit des Zahnbeins.

2. Verwendung eines Anti-Sensibilitätssystems bestehend aus feinverteilten schleifenden und/oder nicht schleifenden Feststoffteilchen und einem wasserlöslichen Anti-Sensibilitätsmittel zur Herstellung einer Mundwasserzusammensetzung, ferner umfassend einen zahnmedizinisch verträglichen Träger oder Vehikulum, zur Behandlung von Überempfindlichkeit des Zahnbeins.

3. Verwendung nach Anspruch 1 oder 2, wobei die feinverteilten Feststoffteilchen nicht schleifend sind und gegebenenfalls schleifende Feststoffteilchen zugemischt sind.

4. Verwendung nach Anspruch 3, wobei die feinverteilten Feststoffteilchen aus nicht schleifendem Siliciumdioxid bestehen.

5. Verwendung nach einem der Ansprüche 2 bis 4, wobei das wasserlösliche Anti-Sensibilitätsmittel ein wasserlösliches Kalium- oder Strontiumsalz oder ein Gemisch davon ist.

6. Verwendung nach Anspruch 5, wobei das Kaliumsalz ausgewählt ist aus Kaliumchlorid, Kaliumnitrat, Kaliumcitrat oder Kaliumacetat.

7. Verwendung nach Anspruch 5, wobei das Strontiumsalz ausgewählt ist aus Strontiumacetat oder Strontiumchlorid.

8. Verwendung nach einem der Ansprüche 1 bis 7, wobei die Zusammensetzung ferner eine Fluoridionenquelle umfaßt.

9. Verwendung nach einem der Ansprüche 1 bis 8, wobei die Zusammensetzung ferner ein Mittel gegen Zahnbelag umfaßt.

10. Verwendung nach einem der Ansprüche 1 bis 9, wobei die Zusammensetzung ferner ein filmbildendes Polymer umfaßt.

11. Verwendung nach einem der Ansprüche 1 bis 10, wobei die Zusammensetzung ferner ein Verdickungsmittel umfaßt.

## Revendications

1. Utilisation d'un système anti-sensibilisant qui consiste en solides particulaires finement divisés abrasifs et/ou non abrasifs dans la fabrication d'une composition d'eau dentifrice comprenant de plus un véhicule ou un support acceptable du point de vue dentaire, pour le traitement d'une hypersensibilité de la dentine.

2. Utilisation d'un système anti-sensibilisant qui consiste en solides particulaires finement divisés abrasifs et/ou non abrasifs et en agent anti-sensibilisant soluble dans l'eau dans la fabrication d'une composition d'eau dentifrice comprenant de plus un véhicule ou un support acceptable du point de vue dentaire, pour le traitement d'une hypersensibilité de la dentine.

3. Utilisation suivant les revendications 1 ou 2, dans laquelle les solides particulaires finement divisés sont non abrasifs, éventuellement mélangés à des solides particulaires qui sont abrasifs.

4. Utilisation suivant la revendication 3, dans laquelle les solides particulaires finement divisés sont une silice non abrasive.

5. Utilisation suivant l'une quelconque des revendications 2 à 4, dans laquelle l'agent anti-sensibilisant soluble dans l'eau est un sel soluble dans l'eau de potassium ou de strontium ou un mélange de ceux-ci.

6. Utilisation suivant la revendication 5, dans laquelle le sel de potassium est choisi parmi le chlorure de potassium, le nitrate de potassium, le citrate de potassium ou l'acétate de potassium.

7. Utilisation suivant la revendication 5, dans laquelle le sel de strontium est choisi parmi l'acétate de strontium ou le chlorure de strontium.

8. Utilisation suivant l'une quelconque des revendications 1 à 7, dans laquelle la composition comprend de plus une source d'ion fluorure.

9. Utilisation suivant l'une quelconque des revendications 1 à 8, dans laquelle la composition comprend de plus un agent contre la plaque dentaire.

10. Utilisation suivant l'une quelconque des revendications 1 à 9, dans laquelle la composition comprend de plus un polymère filmogène.

11. Utilisation suivant l'une quelconque des revendications 1 à 10, dans laquelle la composition comprend de plus un agent épaississant.
